# EUROPEAN PATENT SPECIFICATION

(11) **EP 2 408 368 B1**
(45) Date of publication and mention of the grant of the patent: **29.04.2020**
(21) Application number: 10753278.0
(22) Date of filing: 15.03.2010
(51) Int. Cl.: A61B 5/00, A61B 5/145, A61B 5/1495, C12Q 1/00, A61B 5/1486

(54) **METHOD OF CONTINUOUSLY MEASURING SUBSTANCE CONCENTRATION**
VERFAHREN ZUR DURCHGEHENDEN MESSUNG DER KONZENTRATION EINER SUBSTANZ
PROCÉDÉ DE MESURE CONTINUE DE LA CONCENTRATION D'UNE SUBSTANCE

(30) Priority: 16.03.2009 JP 2009062353; 31.03.2009 US 165062
(43) Date of publication of application: 25.01.2012
(73) Proprietor: ARKRAY, Inc., Minami-ku Kyoto-shi Kyoto 601-8045 (JP)
(72) Inventor: KATSUKI, Koji, Kyoto-shi, Kyoto 602-0008 (JP); TSUKADA, Masashi, Kyoto-shi, Kyoto 602-0008 (JP)
(74) Representative: MacDougall, Alan John Shaw
(86) International application number: PCT/JP2010/001834
(87) International publication number: WO 2010/106781

(56) References cited:
- WO-A1-03/036285
- WO-A1-2005/083413
- WO-A1-2007/013915
- WO-A1-2008/090925
- WO-A2-02/39086
- WO-A2-98/58250
- JP-A- 2000 180 399
- JP-A- 2007 033 459

## Description

### Technical Field

The present invention relates to a method of continuously measuring a substance concentration, such as glucose. In particular, the present invention relates to a method of continuously measuring a blood sugar level, using a glucose sensor implanted in a body.

### Background Art

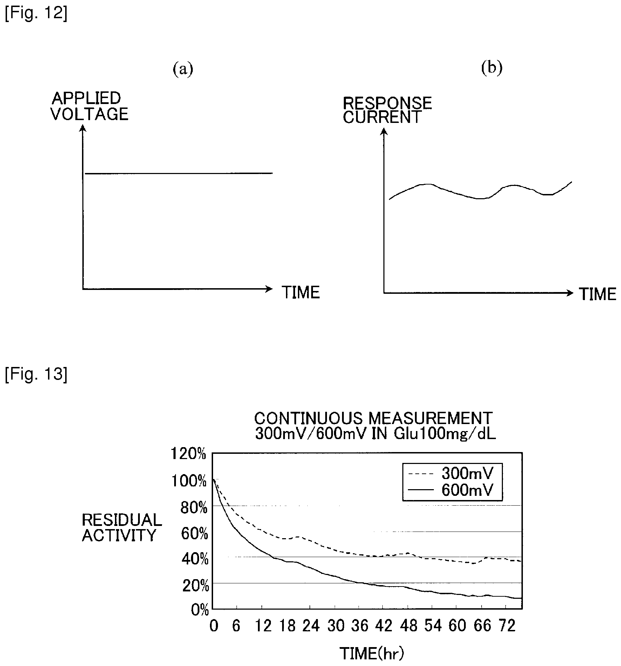

As a method of measuring a blood sugar level, there is a method of continuously measuring a concentration of glucose in interstitial fluid, using a glucose sensor, for example, implanted in the abdomen or the arm of a human body. As the measurement principle, an electrochemical method is mainly adopted. The glucose sensor in this case has at least a working electrode and a counter electrode. At the working electrode, for example, glucose oxidase (GOD) is immobilized. The concentration of glucose is continuously measured based on a response current (see FIG. 12(b)) obtained when a constant voltage of about 0.3 to 0.6 V is continuously applied across the working electrode and the counter electrode (see FIG. 12(a)).

The value of the response current can be obtained, for example, by electrochemically oxidizing hydrogen peroxide generated by a catalytic reaction of GOD (for example, Patent documents 1 and 2), or by using electrons extracted from glucose via GOD with an Os polymer being a mediator (see, for example, Patent document 3).

On the other hand, the glucose concentration is calculated based on a sampling current obtained by sampling a current periodically from the continuously obtained response current values.

However, the response current contains background components attributed to coexisting substances such as ascorbic acid in interstitial fluid or attributed to an external environment such as electromagnetic noise. Therefore, for example, even if measurement data tends to increase (or decrease), it is difficult to ascertain specifically from the response current whether the increase (or decrease) in the measurement data is caused by an increase (or decrease) in the glucose concentration, or whether the increase (or decrease) in the measurement data is caused by an increase (or decrease) in the background components. Therefore, under the environment that is likely to be influenced by the background components, the precision of a signal of the response current obtained from the glucose sensor may be degraded.

Further, it is understood that a glucose oxidoreductase fixed at the working electrode causes protein to be denatured under the application of a voltage, which degrades the stability of a sensor. It is understood that such a degradation in stability depends upon the magnitude of the voltage and the application time of the voltage, as illustrated in FIG. 13 illustrating residual activity in the case of using glucose dehydrogenase (GDH) as the glucose oxidoreductase. More specifically, the stability of the glucose oxidoreductase such as GDH tends to be degraded as the magnitude of the voltage to be applied is larger or the application time of the voltage is longer.

### Citation List

### Patent Literature

PTL 1: Patent JP 2007-516814 A
PTL 2: Patent JP 2007-535991 A
PTL 3: Patent JP 1998-505421 A

Further background art is provided in WO 98/58250 A2, JP 2000 180399 A and WO 2007/013915 A1.

WO 98/58250 A2 discloses a method of calibrating an analyte sensor *in vivo,* which involves providing a supply of the analyte to the subject in the vicinity of the sensor, preferably as a soluble coating on the sensor, so as to saturate the sensor and obtain a maximum signal, causing the analyte in the vicinity of the sensor to be consumed so as to obtain a base line signal, and calculating a calibration function to translate the measured saturation and base line signals to corresponding points on a laboratory calibrated reference curve. This calibration function is subsequently used to translate a measured point on the actual *in vivo* performance curve of the sensor to the corresponding point on the laboratory calibrated reference curve, to thereby obtain a calibrated output for the sensor *in vivo* taking account of the conditions found in use.

JP 2000 180399 A discloses a determination method of a substrate, in which an electrode potential E1 is applied after a constant time has elapsed after the contact of a sample with a reaction layer to obtain a background current and, further, after a constant time has elapsed from the application of potential E1, a response current at potential E2 satisfying a condition of E2>E1 is obtained, and the substrate is determined on the basis of the difference between the response current and the background current.

WO 2007/013915 A1 discloses a sensor system, device, and methods for determining the concentration of an analyte in a sample, involving gated amperometric pulse sequences including multiple duty cycles of sequential excitations and relaxations.

### Summary of Invention

The present invention is a method of continuously measuring a substance concentration as defined in Claim 1 of the appended claims.

### Technical Problem

It is an object of the present invention to improve the measurement precision and the stability of a sensor to be used for measurement in continuous measurement of a substance such as glucose.

### Solution to Problem

The present invention provides a method of continuously measuring a substance concentration based on a response when a voltage is applied to a sensor, including: a response voltage application step of applying a response voltage at which a current attributed to the substance is obtained; a non-response voltage application step of applying a non-response voltage at which a current attributed to a background component is obtained; a comparing step of comparing the current obtained in the response voltage application step with a first threshold value, or comparing the current obtained in the non-response voltage application step with a second threshold value; and a determining step of determining whether or not to calculate the substance concentration based on the result of the comparing step.

The response voltage application step and the non-response voltage application step, and the respective obtaining of the current attributed to the substance and the current attributed to the background component, are performed alternately and repeatedly, with the response voltage transitioning to the non-response voltage without interruption, and the non-response voltage transitioning to the response voltage without interruption.

Each of the response voltage application step and the non-response voltage application step does not necessarily need to be conducted by applying a single pulse at a constant voltage, and may be conducted, for example, by applying a stepped pulse composed of a combination of a plurality of pulses of a constant voltage with different potentials.

In the present invention, for example, the substance concentration is calculated based on a difference between the current obtained in the response voltage application step and the current obtained in the non-response voltage application step. In this case, when a voltage to be applied to a sensor is increased, the voltage applied in the non-response voltage application step is set in a range of -0.5 to +0.5 V, preferably in a range of -25 to +25 mV with respect to an applied voltage (reaction potential) at which the current attributed to the substance starts being obtained.

The present invention may further include a standby step of applying a voltage smaller in absolute value than the non-response voltage after performing the response voltage application step and the non-response voltage application step alternately a predetermined number of times. The standby step may be performed by setting a circuit including the sensor to become an open circuit.

The sensor is implanted, for example, in a body for use. The sensor may include an enzyme-immobilized electrode. Needless to say, the present invention may be applied to the case of measuring the concentration of a substance such as glucose in blood or interstitial fluid extracted out of a body or the case of measuring a substance in substance-containing fluid other than blood and interstitial fluid or fluid outside of a human body.

The substance is, for example, glucose, and the sensor is, for example, a glucose sensor. The glucose sensor may include an electrode with a glucose oxidoreductase immobilized thereon.

### Brief Description of Drawings

[fig.1]FIG. 1 is a cross-sectional view illustrating an example of a glucose continuous measurement apparatus for carrying out a continuous measurement method of glucose.
[fig.2]FIG. 2 is an entire perspective view illustrating a glucose sensor in the glucose continuous measurement apparatus illustrated in FIG. 1, together with a main portion enlarged view.
[fig.3]FIG. 3 is a block diagram illustrating a schematic configuration of the glucose continuous measurement apparatus illustrated in FIG. 1.
[fig.4]FIG. 4 is a graph illustrating an example of a voltage application pattern with respect to the glucose sensor illustrated in FIG. 2.
[fig.5]FIG. 5 is a graph illustrating an example of a response current of when a voltage is applied in the pattern illustrated in FIG. 4.
[fig.6]FIG. 6 is a graph illustrating measurement results of voltammetry with respect to a plurality of glucose concentrations.
[fig.7]FIGS. 7 are graphs each illustrating another example of an application pulse of a reaction voltage and a non-reaction voltage in one step.
[fig.8]FIG. 8 is a graph illustrating measurement results of voltammetry in Example 1.
[fig.9]FIGS. 9(a) and 9(b) are graphs illustrating measurement results of a change with time of a response current in Example 1.
[fig.10]FIG. 10 is a graph illustrating measurement results of response characteristics in Example 1.
[fig.11]FIG. 11 is a graph illustrating evaluation results of stability in Example 2.
[fig.12]FIG. 12(a) is a graph illustrating a pattern of voltage application with respect to a glucose sensor in a conventional example, and FIG. 12(b) is a graph illustrating an example of a response current with respect to the voltage application illustrated in FIG. 12(a).
[fig.13]FIG. 13 is a graph illustrating a change with time of residual activity of enzyme in the conventional example.
[fig.14]FIG. 14 is a graph illustrating a change in current density in the case of applying a stepped pulse voltage as illustrated in FIG. 7(a) repeatedly to the enzyme-immobilized electrode.
[fig.15]FIG. 15 is a graph illustrating a relationship between the current density and the glucose concentration.
[fig. 16]FIG. 16 is a graph illustrating measurement results of a response current in the case of applying a pulse voltage repeatedly to the enzyme-immobilized electrode.
[fig. 17]FIG. 17 is a graph illustrating measurement results of a response current in the case of applying a constant voltage of 600 mV continuously to the enzyme-immobilized electrode.
[fig. 18]FIG. 18 is a graph illustrating evaluation results of stability in Example 4.
[fig. 19]FIG. 19 is a graph illustrating a change in response current due to an increasing glucose concentration in the case of applying a constant voltage continuously to the enzyme-immobilized electrode.
[fig.20]FIG. 20 is a graph illustrating a change in response current due to an increasing glucose concentration in the case of applying a pulse voltage repeatedly to the enzyme-immobilized electrode.
[fig.21]FIG. 21 is a graph illustrating the response current 13 in the case of applying a constant voltage continuously to the enzyme-immobilized electrode, and the response current I4 in the case of applying a pulse voltage repeatedly to the enzyme-immobilized electrode.

### Description of Embodiments

Hereinafter, a method of substance continuous measurement according to the present invention is described with reference to the drawings, illustrating a case of measuring a glucose concentration in body fluid.

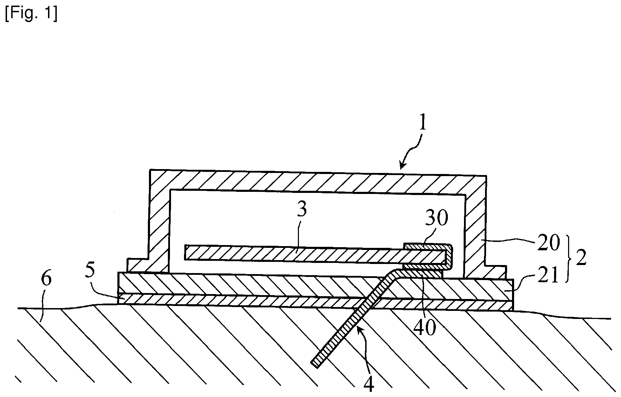

A glucose continuous measurement apparatus 1 illustrated in FIG. 1 is capable of continuously measuring the concentration of glucose in blood or interstitial fluid, and is attached for use to the abdomen or the shoulder skin of a human body. The glucose continuous measurement apparatus 1 includes a housing 2, a circuit board 3, and a glucose sensor 4.

The housing 2 forms an outer shape of the glucose continuous measurement apparatus 1, and includes a cover 20 and a base 21. The cover 20 and the base 21 contain the circuit board 3 in a space defined by the cover 20 and the base 21, and are fixed to each other. The housing 2 preferably has a waterproof property or water resistance. In the housing 2, for example, at least the cover 20 (base 21 if required) is formed of a material with very low water permeability such as metal or polypropylene resin.

The base 21 is a portion through which the glucose sensor 4 is inserted, and an end 40 of the glucose sensor 4 is fixed on the base 21. An adhesive film 5 is fixed on the base 21. The adhesive film 5 is used when the glucose continuous measurement apparatus 1 is fixed to the skin. As the adhesive film 5, a tape having adhesion on its both surfaces is used.

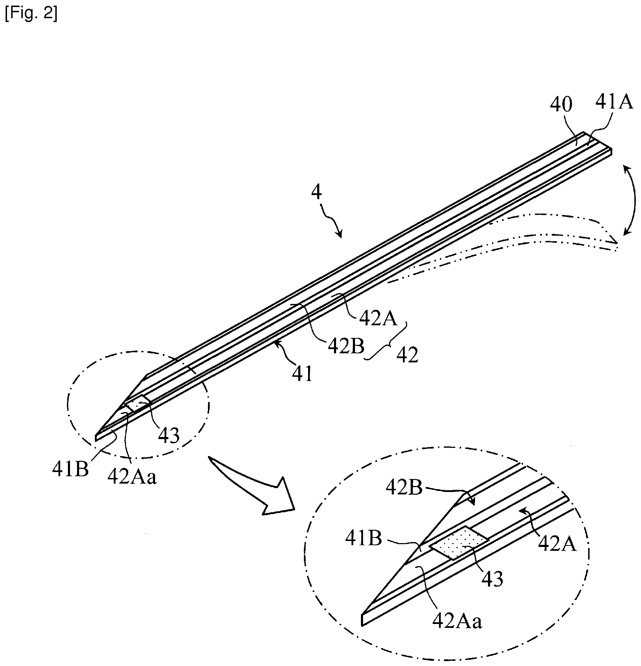

The circuit board 3 is equipped with electronic components required for predetermined operations (for example, the application of a voltage, the arithmetic operation of a glucose concentration, or the communication with the outside) of the glucose continuous measurement apparatus 1. The circuit board 3 further includes a terminal 30 for coming into contact with an electrode 42 of the glucose sensor 4 (see FIG. 2) described later. The terminal 30 is used for applying a voltage to the glucose sensor 4 and obtaining a response current value from the glucose sensor 4.

The glucose sensor 4 is used for obtaining a response in accordance with a glucose concentration in blood or interstitial fluid. In the glucose sensor 4, the end 40 protrudes from a skin 6 and is in contact with the terminal 30 of the circuit board 3, and the most remaining part thereof is inserted in the skin 6.

As illustrated in FIG. 2, the glucose sensor 4 includes a base 41, the electrode 42, and an immobilized enzyme part 43.

The base 41 supports the electrode 42 and is formed in a sheet shape having insulation and flexibility. In the base 41, an end 41A is present in the housing 2 while an end 41B is formed so as to be sharp. If the end 10B is configured so as to be sharp, the glucose sensor 4 can be inserted in the skin 6 easily, which can alleviate the pain to a user.

As a material for the base 40, any material having appropriate insulation without causing any damage to a human body can be used, and for example, thermoplastic resin such as PET (Polyethylene Terephthalate), PP (polypropylene), or PE (polyethylene), or thermosetting resin such as polyimide resin or epoxy resin can be used.

The electrode 42 is used for applying a voltage to the immobilized enzyme part 43 and extracting electrons from the immobilized enzyme part 43. The electrode 42 includes a working electrode 42A and a counter electrode 42B. The working electrode 42A transfers electrons with respect to glucose. The counter electrode 42B is used for applying a voltage together with the working electrode 42A. The electrode 42 can be formed by screen printing using carbon ink.

The immobilized enzyme part 43 mediates the transfer of electrons between glucose and the working electrode 42A. The immobilized enzyme part 43 is formed by immobilizing glucose oxidoreductase at an end 42Aa of the working electrode 42A.

As the glucose oxidoreductase, glucose oxidase (GOD) and glucose dehydrogenase (GDH) can be used. As a method of immobilizing the glucose oxidoreductase, various known methods, for example, methods using a polymer such as polymerizable gel, polyacrylamide or phosphorus, an MPC polymer in which a silane coupling agent is introduced into a phospholipid polymer, or a protein membrane can be adopted.

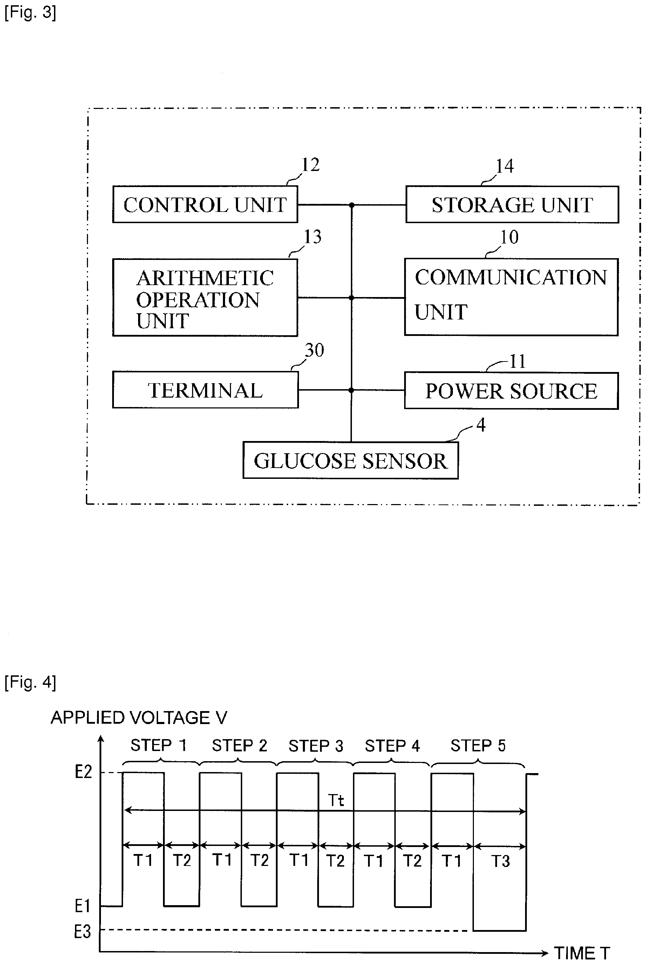

As illustrated in FIG. 3, the glucose continuous measurement apparatus 1 further includes a communication unit 10, a power source 11, a control unit 12, an arithmetic operation unit 13, and a storage unit 14, in addition to the circuit board 3 and the glucose sensor 4.

The communication unit 10 is used for data communication between the glucose continuous measurement apparatus 1 and an external information processing terminal. The communication unit 10 includes at least a transmitting unit, and includes a receiving unit if required.

For data communication, for example, wireless communication means (IrDA using infrared radiation or Bluetooth using a frequency band of 2.4 GHz) can be used. Needless to say, wired data communication may be performed by connecting the communication unit 10 of the glucose continuous measurement apparatus 1 to a communication unit of the external information processing terminal with a cable or the like.

An example of the external information processing terminal includes an insulin supply apparatus for administering insulin to a human body, a simple blood sugar level measurement apparatus, a watch-type display, or a personal computer.

The data communication between the glucose continuous measurement apparatus 1 and the insulin supply apparatus is performed, for example, by sending measurement results of a glucose concentration in the glucose continuous measurement apparatus 1 to the insulin supply apparatus. This enables an insulin amount to be administered to a human body to be controlled based on the measurement data from the glucose continuous measurement apparatus 1.

The data communication between the glucose continuous measurement apparatus 1 and the simple blood sugar level measurement apparatus is performed, for example, by sending blood sugar level measurement results in the simple blood sugar level measurement apparatus to the glucose continuous measurement apparatus 1. Thus, the measurement results in the glucose continuous measurement apparatus 1 are compared with the measurement results in the simple blood sugar level measurement apparatus, and if there is a deviation by a predetermined value or more is present between those measurement results, the glucose continuous measurement apparatus 1 can be calibrated. Further, raw data (response current) measured in the glucose continuous measurement apparatus 1 may be sent to the simple blood sugar level measurement apparatus.

The data communication between the glucose continuous measurement apparatus 1 and the watch-type display is performed, for example, by sending the blood sugar level measurement results in the glucose continuous measurement apparatus 1 to the watch-type display. Consequently, even when the glucose continuous measurement apparatus 1 is attached to a position such as the shoulder, which a user has difficulty in visually recognizing, the measurement results in the glucose continuous measurement apparatus 1 can be displayed on the watch-type display so that the user can grasp the measurement results.

The data communication between the glucose continuous measurement apparatus 1 and a personal computer is performed, for example, by sending blood sugar level measurement results or raw data (response current) in the glucose continuous measurement apparatus 1 to the personal computer. This allows the transition of a glucose concentration to be monitored on the personal computer. Further, data for calibration from the personal computer may be sent to the glucose continuous measurement apparatus 1.

The power source 11 is a DC power source for supplying power to the circuit board 3 and the glucose sensor 4. As the power source 11, for example, a button battery with a power supply voltage of 1 to 3 V is used.

The control unit 12 controls various operations such as the control of a timing of voltage application and an applied voltage value, the sampling of a response current, the arithmetic operation of a glucose concentration, and the communication with the external information processing terminal.

The arithmetic operation unit 13 performs various arithmetic operations such as those required for the operation of the glucose continuous measurement apparatus 1, in addition to the arithmetic operation of a glucose concentration, for example.

The storage unit 14 stores programs and data (for example, data on a calibration curve, correction data, or data on a voltage application pattern) required for various arithmetic operations. The storage unit 14 may further store a response current value from the glucose sensor 4 and a glucose concentration obtained by an arithmetic operation.

The control unit 12, the arithmetic operation unit 13, and the storage unit 14 are realized by electronic components mounted on the circuit board 3, such as a CPU (or an MPU), a ROM, and a RAM.

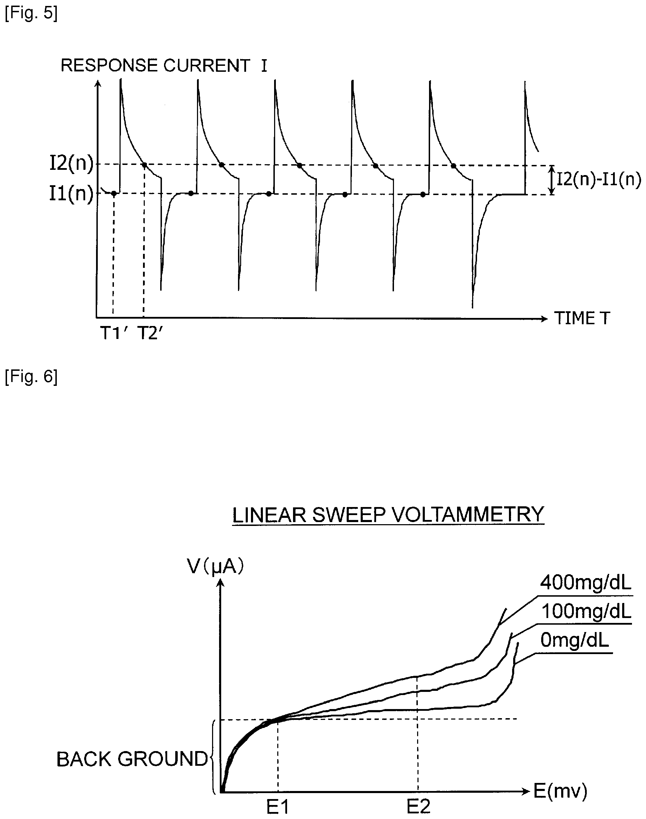

Here, the application of a voltage to the glucose sensor 4 in the glucose continuous measurement apparatus 1 is performed, for example, in a pattern illustrated in FIG. 4. In the illustrated example, assuming a combination of a state in which a non-reaction voltage E1 is applied for a predetermined time T2 and a state in which a reaction voltage E2 is applied for a predetermined time T1 as one step (one pulse), the step is performed repeatedly, and a standby time T3 is set after a plurality of the steps. Further, FIG. 5 illustrates an example of a response current in the case of the voltage application pattern illustrated in FIG. 4.

The non-reaction voltage E1 is a voltage at which glucose does not react at all or at which glucose reacts so slightly as not to react substantially (see FIG. 6). More specifically, a response current obtained when the non-reaction voltage E1 is applied to the glucose sensor 4 contains a very small response amount attributed to glucose, and is mainly attributed to a background (sensitivity) peculiar to the sensor, a background attributed to coexisting substances, and a background attributed to external environment such as electromagnetic noise.

The magnitude of the non-reaction voltage E1 is set in accordance with the specifications (for example, the amount of enzyme to be used, an immobilizing method, constituent materials for electrodes, or a reaction area) of the glucose sensor 4. Specifically, the non-reaction voltage E1 is set at a voltage (reaction potential) at which a response current attributed to glucose starts flowing when a voltage applied to the glucose sensor 4 is increased or in a range of -0.5 to +0.5 V (preferably -25 to +25 mV) from the reaction potential. In the illustrated glucose sensor 4, the non-reaction voltage E1 is set at, for example, -1 to 1 V. Further, the application time T2 of the non-reaction voltage E1 in one step is set at a time required for a response current to be stabilized to a constant value, e.g., 0.1 to 300 sec.

The reaction voltage E2 is a potential at which glucose reacts with sufficient resolution (see FIG. 6). The magnitude of the reaction voltage E2 is set in accordance with the specifications (for example, the amount of enzyme to be used, an immobilizing method, constituent materials for electrodes, or a reaction area) of the glucose sensor 4, and is set at, for example, -1 to 1 V. Further, the application time T1 of the reaction voltage E2 in one step is set at a time required for a rapid change of a response current to disappear, for example, 0.1 to 300 micro sec.

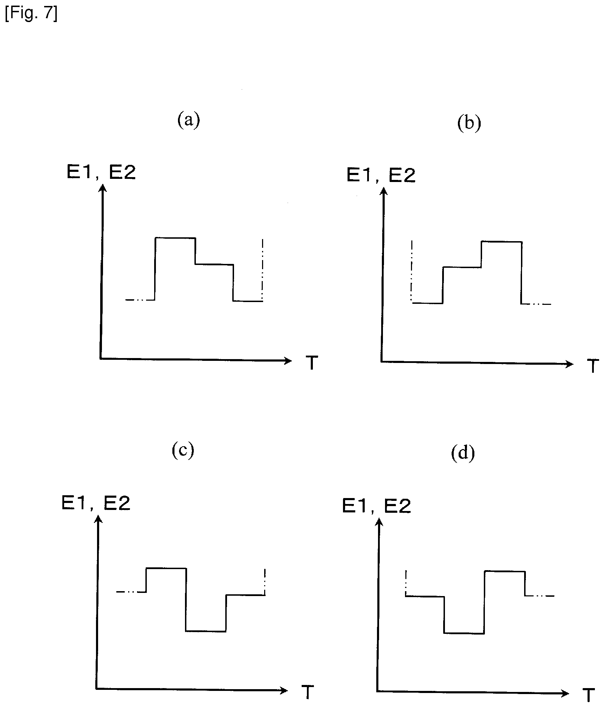

The non-reaction voltage E1 and the reaction voltage E2 do not necessarily need to be applied to the glucose sensor 4 as a single pulse of a constant voltage in one step as illustrated in FIG. 4, and may be applied to the glucose sensor 4, for example, as a stepped pulse of a combination of a plurality of pulses of constant voltages with different potentials as illustrated in FIGS. 7(a) to 7(d). In the case where the non-reaction voltage E1 and the reaction voltage E2 are applied as a stepped pulse in one step, the number of constant voltages may be two or four or more although the number of constant voltages is three in FIGS. 7(a) to 7(d).

The standby time T3 corresponds to the state in which a standby voltage E3 is applied as a voltage applied to the glucose sensor 4, which is further smaller than the non-reaction voltage E1 in absolute value. The standby time T3 is for preventing an unnecessarily large voltage from being applied to the glucose sensor 4, and is set, for example, at 0.1 to 300 sec. The application voltage (standby voltage) E3 during the standby time T3 is set at 0 V or more and less than the non-reaction voltage E1 in absolute value. Here, as a method of setting the standby voltage E3 at 0 V, there is a method of turning off a main power source, in addition to a method of setting the voltage applied to the glucose sensor 4 at 0 V while applying a driving voltage to the circuit board 3. Needless to say, the standby time T3 is not necessarily required, and further, the standby time T3 may be provided arbitrarily after one step or a plurality of steps (for example, 2 to 1,500 steps) including the non-reaction voltage E1 and the reaction voltage E2.

In the application voltage pattern as described above, during the arithmetic operation of a glucose concentration, a response current I2(n) is sampled at the reaction voltage E2, while a response current I1(n), during the application of a non-reaction voltage E1 in the same step as the step during which the response current I2(n) is sampled, is sampled. Respective sampling timings T'1 and T'2 for current values obtained during the application of the voltages E1 and E2 are provided to a state in which a fluctuation of a response current is small and a response current is relatively stabilized.

It should be noted that impedance, energy, or quantity of electricity, for example, may be obtained as a response in place of sampling of a response current.

On the other hand, in the arithmetic operation unit 13, a glucose concentration is calculated considering a response current sampled during the application of the non-reaction voltage E1. Typically, a glucose concentration is arithmetically operated from a response current value (I2(n)-I1(n)) obtained by subtracting the response current I1(n) at the non-reaction voltage E1 from the response current I2(n) at the reaction voltage E2.

An glucose concentration may be calculated once in one cycle including a plurality of steps, or may be performed in one cycle selected from a plurality of cycles. Further, in one cycle, a glucose concentration may be subjected to arithmetic operation for each step to determine an average value of the arithmetic operation results as a glucose concentration of that step.

Further, a threshold value of a measured value or a threshold value regarding the difference from that at the previous measurement may be set regarding the response current I2(n) at the reaction voltage E2, the response current I1(n) at the non-reaction voltage E1, or a difference value (I2(n)-I1(n)) of the response current values, and comparison may be made with respect to the threshold value before the arithmetic operation of a glucose concentration. This makes it possible to determine whether each parameter is an appropriate value to be used for an arithmetic operation or determine whether or not to perform an arithmetic operation of a glucose concentration (measurement of glucose). Consequently, in the glucose continuous measurement apparatus 1, the quantification of glucose with higher precision can be performed.

The glucose concentration obtained by the arithmetic operation is used mainly for determining the amount of administration of insulin and calibrating the glucose continuous measurement apparatus 1 if required. For the arithmetic operation of a glucose concentration for calibration, it is preferred to adopt an average value of a glucose concentration calculated for each step selected arbitrarily from each of a plurality of cycles.

As described above, a response current with respect to the non-reaction voltage E1 is not related to or not substantially related to a glucose reaction, and is mainly attributed to background components. Therefore, a glucose concentration can be calculated while minimizing the influence of the background components if a response current corresponding to the non-reaction voltage E1 is considered, to thereby enhance measurement precision.

Further, in a pattern in which the non-reaction voltage E1 and the reaction voltage E2 are applied to the glucose sensor 4 repeatedly as a voltage application pattern, compared with the case where a constant voltage is applied continuously, a voltage (energy) to be applied to the glucose sensor 4 per unit time is decreased. Therefore, the degradation caused by the denaturation and the like of glucose oxidoreductase can be suppressed, and hence, the sensitivity of the glucose sensor 4 can be stabilized over a long period of time and the life of the glucose sensor 4 can be prolonged. Consequently, the replacement frequency of the glucose sensor 4 and the calibration frequency of the glucose continuous measurement apparatus 1 can be reduced, which alleviates an operational burden and an economical burden on a user. Further, if a voltage (energy) to be applied to the glucose sensor 4 can be decreased, the power consumption in the case of measuring glucose continuously can be reduced, and hence, the life of the battery can be prolonged. As a result, in the case of using a disposable battery such as a button battery as a battery, the economical burden on the user can be alleviated. In particular, if the standby time T3 is provided and the power source is turned off during the standby time T3, the life of the battery can be prolonged securely.

According to the glucose continuous measurement apparatus 1 described above, the arithmetic operation function of a glucose concentration is incorporated in the glucose continuous measurement apparatus 1, but the external information processing terminal may be allowed to calculate a glucose concentration while a response value such as a response current value is measured in the glucose continuous measurement apparatus 1.

Further, in the above-mentioned embodiment, the case where the reaction voltage E2 is applied after the non-reaction voltage E1 is applied is defined as one cycle (one pulse). However, the case where the non-reaction voltage E1 is applied after the reaction voltage E2 is applied may be defined as one cycle (one pulse).

The present invention can be applied to the case of using a substance other than glucose or substance-containing fluid other than body fluid, as well as to the case of measuring a glucose concentration in body fluid of a human body.

Next, the effects of the present invention are studied as examples.

### Example 1

In this example, response characteristics of when a pulse voltage is applied repeatedly to an enzyme-immobilized electrode were studied.

### (Production of enzyme-immobilized electrode)

An enzyme-immobilized electrode was produced by immobilizing GDH on the surface of a carbon electrode via a phospholipid polymer. For producing the carbon electrode, first, 100 micro liter of liquid paraffin was added to powder mixed thoroughly with 120 mg of Ketjen Black (produced by Lion Corporation), followed by mixing well to form a material paste. The material paste was packed into a base electrode for producing a paste electrode with an area of 3 phi (produced by BAS Inc.), and compressed with a JURAN connecting rod to obtain a carbon electrode. As the phospholipid polymer, an MPC polymer (trade name: "LIPIDURE", produced by NOF Corporation) with tetraethoxysilane introduced thereinto as a silane coupling agent was used. Enzyme immobilization was performed by soaking the carbon electrode modified with the phospholipid in 1,250 U/mL of a GDH solution.

### (Setting of a voltage to be applied)

A voltage to be applied was determined based on results obtained by measuring voltage dependency of a glucose response by voltammetry, using an enzyme-immobilized electrode produced previously.

The glucose concentration was set at 600 mg/dL in voltammetry.

As an electrode system in voltammetry, the enzyme-immobilized electrode produced previously as a working electrode, a platinum electrode as a counter electrode, and a silver/silver chloride electrode as a reference electrode were used.

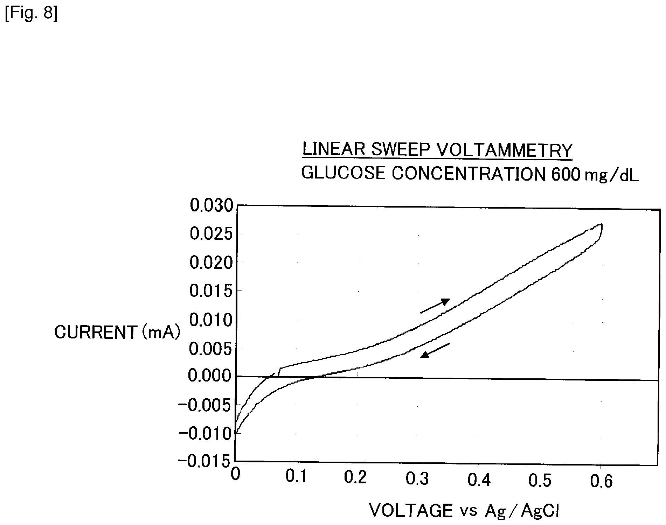

The sweep in voltammetry was conducted in a positive direction and a negative direction, respectively, setting a sweep speed at 0.1 V/sec in a range of 0 to 0.6 V. FIG. 8 illustrates measurement results of voltammetry.

As seen from FIG. 8, it is understood that an oxidation current responding to glucose is detected at an applied voltage of around 75 mV. From this result, regarding the non-response voltage E1 that does not cause a glucose reaction or does not substantially cause a glucose reaction, a constant voltage of 100 mV was set considering a margin in the following study. On the other hand, regarding the reaction voltage E2 exhibiting a glucose response, a constant voltage of 700 mV was set, assuming that the potential difference from the non-response voltage E1 was set at 600 mV.

### (Pattern of an applied voltage)

The application times T1 and T2 of the non-response voltage E1 and the response voltage E2 were set at 10 seconds.

The standby time T3 was set at 40 seconds. It should be noted that the standby time T3 was set so that an electrode system including an enzyme-immobilized electrode became an open circuit (0 V) every four steps (four pulses).

### (Measurement of response characteristics of glucose)

The response characteristics were measured by adding glucose at a particular timing while rotating a stirring bar with a stirrer under the condition that an enzyme-immobilized electrode (working electrode), a platinum electrode (counter electrode), and a silver/silver chloride electrode (reference electrode) were soaked in a container containing the stirring bar together with water.

Glucose was added during the standby time T3 in the fourth cycle, with four steps (four pulses) being one cycle. Glucose was added three times, and the concentration of glucose was changed successively from 0 mg/dL, to 50 mg/dL, 100 mg/dL, and 600 mg/dL.

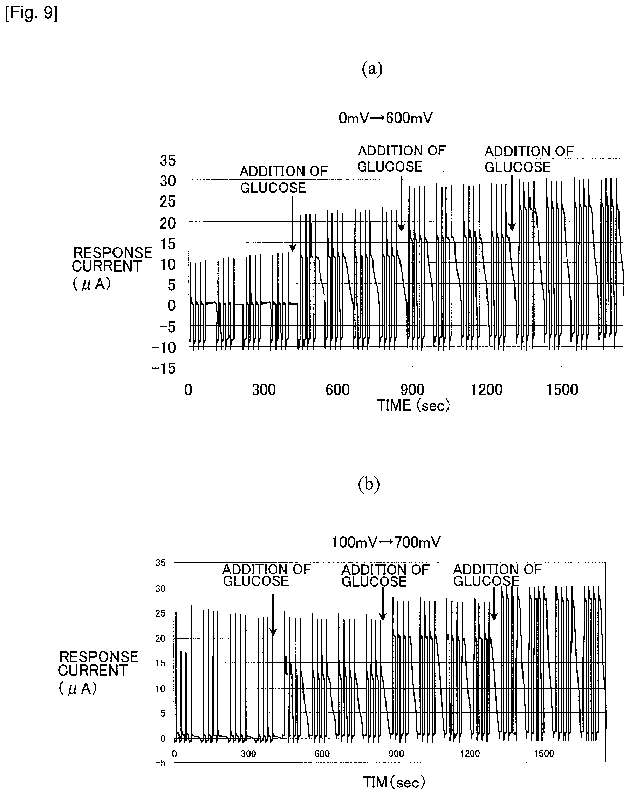

A response current was measured, setting the non-reaction voltage E1 and the reaction voltage E2 at constant voltages of 0 mV and 600 mV, respectively. FIG. 9(a) illustrates measurement results of the response current. For reference, FIG. 9(b) illustrates the case where a response current was measured, setting the non-reaction voltage E1 and the reaction voltage E2 at 100 mV and 700 mV, respectively.

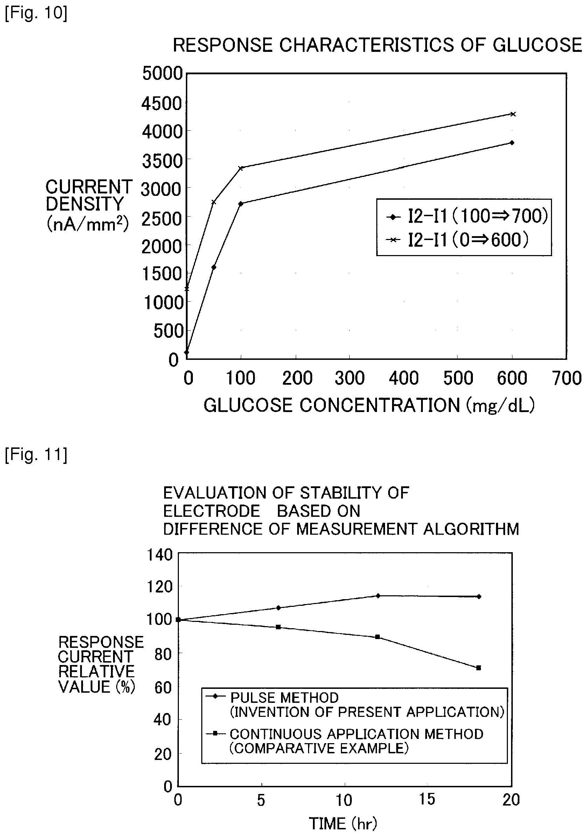

Further, the current values I1 and 12 after 10 seconds of the application of the non-response voltage E1 and the response voltage E2 were sampled, respectively, and FIG. 10 illustrates a relationship between a difference value between the current values I1 and I2 and the glucose concentration. In FIG. 10, the difference value of the response currents is illustrated as a value (current density) obtained by dividing by the area of the enzyme-immobilized electrode.

As seen from FIGS. 9(a) and 9(b), it was confirmed that a current value increased due to the addition of glucose. More specifically, it was confirmed that glucose reacted to the response voltage E2 to thereby generate a response current.

Further, in the case where the non-response voltage E1 was set at 100 mV when an applied voltage was decreased from the response voltage E2 to the non-response voltage E1, a fluctuation amount (background) of a response current became remarkably smaller, compared with the case of setting the non-response voltage E1 at 0 mV.

On the other hand, as illustrated in FIG. 10, in the case where the non-response voltage E1 was set at 100 mV, compared with the case of setting the non-response voltage E2 at 0 mV, the difference (12-11) between the response current 12 during the application of the response voltage E2 and the response current I1 during the application of the non-response voltage E1 decreases irrespective of the glucose concentration. It was also understood from this point that, in the case of setting the non-response voltage E1 at 100 mV, i.e., in the case of setting the non-response voltage E1 to be larger than 0V, the influence by background is small and response sensitivity is high.

### Example 2

In this example, stability of an enzyme-immobilized electrode when a pulse voltage is applied repeatedly to the enzyme-immobilized electrode was evaluated.

### (Production of enzyme-immobilized electrode)

The enzyme-immobilized electrode was produced by immobilizing GDH on the surface of a carbon electrode via a phospholipid polymer. The carbon electrode was formed on the surface of a polyimide base material. As a material for the electrode, printing ink containing a mixture of 40wt% of Ketjen Black (produced by Lion Corporation), 40wt% of polyester resin as a binder, and 20wt% of isophorone as a solvent was used. The printing ink was printed onto the surface of the polyimide base material to a thickness of 10 micro meters, and dried under environment of 150 deg C for 30 minutes to obtain an electrode. As the phospholipid polymer, an MPC polymer (trade name: "LIPIDURE", produced by NOF Corporation) with tetraethoxysilane introduced thereinto as a silane coupling agent was used. Enzyme immobilization was performed by soaking the carbon electrode modified with the phospholipid in 1,250 U/mL of a GDH solution.

### (Evaluation of stability)

The stability was evaluated as the sensitivity of the enzyme-immobilized electrode after a response current was measured continuously using 600 mg/dL of a glucose solution.

The electrode system and the applied voltage pattern were set to be the same as those in Example 1. It should be noted that the non-response voltage E1 and the response voltage E2 were set at 100 mV and 700 mV, respectively.

The voltage application time was set at 18 hours in total, and the sensitivity of the enzyme-immobilized electrode was measured every 6 hours as a response current. For comparison, the sensitivity in the case of applying a constant voltage of 600 mV continuously was evaluated. FIG. 11 illustrates the sensitivity in these measurement methods as relative values of response currents with respect to initial sensitivity (0-hour response current).

As seen from FIG. 11, according to the method of applying a constant voltage continuously as a comparative example, a response current was decreased with time, and thus, the degradation in an enzyme electrode was observed. In contrast, according to a method of applying a pulse voltage repeatedly, the decrease in a response current with time was not observed, and the stability of the enzyme-immobilized electrode was confirmed to be satisfactory. In this respect, GDH is denatured and deteriorated by application of the voltage, but a response voltage application time in the method of applying a pulse voltage repeatedly totally becomes shorter as compared to the method of applying a constant voltage continuously in the same measurement time. Accordingly, it is understood that the shorter response voltage application time suppresses GDH from being deteriorated, and maintains the stability of GDH.

### Example 3

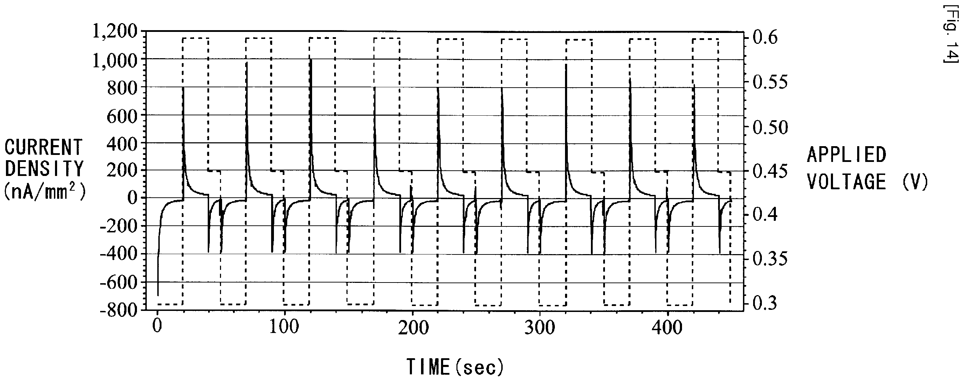

In Example 3, studies were made on a performance evaluation of a stepped pulse voltage. The production of an enzyme-immobilized electrode, and an electrode system are the same as those in Example 1. FIG. 14 is a graph illustrating a change in current density in the case of applying a stepped pulse voltage as illustrated in FIG. 7(a) repeatedly to the enzyme-immobilized electrode. The current density is a value calculated by dividing a response current value by the area of the enzyme-immobilized electrode. In FIG. 14, the stepped pulse voltage is represented by a dashed line, and the current density is represented by a solid line. One pulse is assumed to be a combination of a state where the non-response voltage E1 is applied, a state where the response voltage E2 is applied, and a state where the intermediate voltage is applied. For the voltage to be applied, the non-response voltage E1 was set at a constant voltage of 300 mV, the response voltage E2 was set at a constant voltage of 600 mV, and the intermediate voltage was set at a constant voltage of 450 mV.

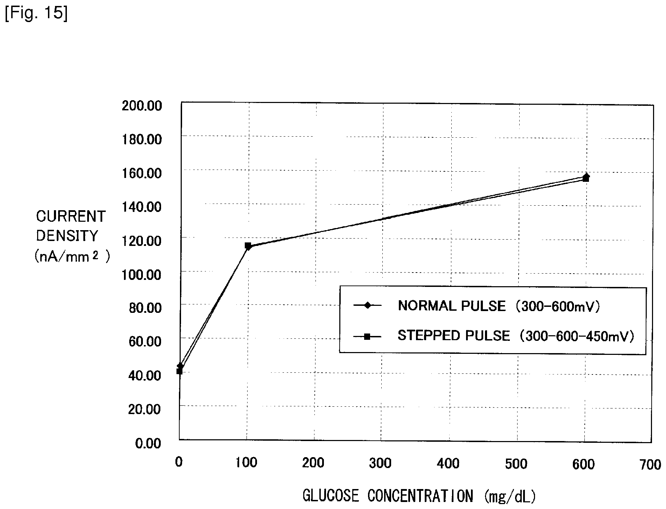

FIG. 15 is a graph illustrating a relationship between the current density and the glucose concentration. Each of the response current I1 obtained after application of the non-response voltage E1 and the response current I2 obtained after application of the response voltage E2 was sampled to calculate a difference value between the response current I1 and the response current 12. The current density is a value calculated by dividing the difference value between the response currents by the area of the enzyme-immobilized electrode.

FIG. 15 illustrates, as the stepped pulse voltage, a pulse voltage assuming a combination of a state where the non-response voltage E1 is applied, a state where the response voltage E2 is applied, and a state where the intermediate voltage is applied as one pulse. For the voltage to be applied, the non-response voltage E1 was set at a constant voltage of 300 mV, the response voltage E2 was set at a constant voltage of 600 mV, and the intermediate voltage was set at a constant voltage of 450 mV. FIG. 15 also illustrates, as a normal pulse voltage, a pulse voltage assuming a combination of a state where the non-response voltage E1 is applied and a state where the response voltage E2 is applied as one pulse. For the voltage to be applied, the non-response voltage E1 was set at a constant voltage of 300 mV, and the response voltage E2 was set at a constant voltage of 600 mV.

As illustrated in FIG. 15, both the stepped pulse voltage and the normal pulse voltage exhibit an increased glucose concentration and an increased current density. Thus, it was confirmed that the stepped pulse voltage had almost the same performance as compared to the normal pulse voltage.

### Example 4

In Example 4, in the case of using GOD as a glucose oxidoreductase, studies were made on response characteristics in the case of applying a pulse voltage repeatedly to the enzyme-immobilized electrode.

### (Production of enzyme-immobilized electrode)

Platinum was deposited on the surface of a polyimide base material by a sputtering method to a thickness of 30 nm, to thereby form a conductive layer on the surface of the polyimide base material. Then, the conductive layer was soaked in a GOD solution (1 mg/mL GOD aqueous solution) to form a GOD layer on the conductive layer. Thus, an enzyme-immobilized electrode was produced. The concentration of GOD in the GOD solution was 1,100 U/mL in terms of activity.

### (Measurement of response characteristics of glucose)

Response characteristics of glucose were measured by adding glucose while allowing a stirring bar to rotate with a stirrer in a state where an enzyme-immobilized electrode (working electrode), a platinum electrode (counter electrode), and a silver/silver chloride electrode (reference electrode) were soaked in a container containing a phosphate buffer (100 mM, pH=7.0) and the stirring bar. The concentration of glucose was changed from 0 mg/dL to 600 mg/dL by adding glucose once.

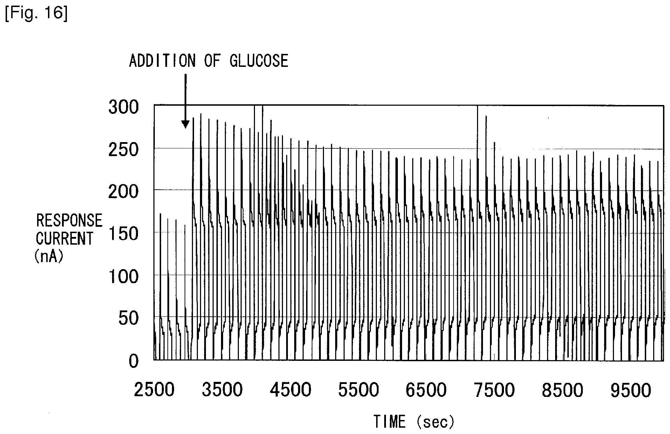

FIG. 16 is a graph illustrating measurement results of a response current in the case of applying a pulse voltage repeatedly to the enzyme-immobilized electrode. For the voltage to be applied, the non-response voltage E1 was set at a constant voltage of 300 mV, and the response voltage E2 was set at a constant voltage of 600 mV. For the applied voltage pattern, the application time T2 of the non-response voltage E1 was set at 30 seconds, and the application time T1 of the response voltage E2 was set at 30 seconds. The standby time T3 was set at 0 second. As illustrated in FIG. 16, it was confirmed that, in the case of applying a pulse voltage repeatedly to the enzyme-immobilized electrode, the response current was kept at an almost constant value.

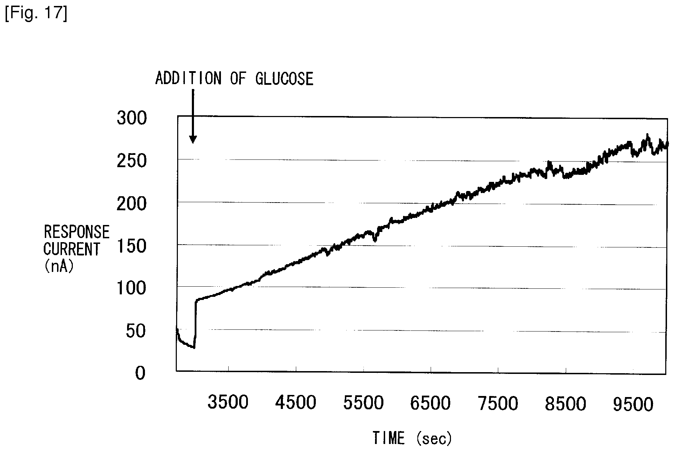

For comparison, FIG. 17 illustrates measurement results of a response current in the case of applying a constant voltage of 600 mV continuously to the enzyme-immobilized electrode. As illustrated in FIG. 17, it was confirmed that, in the case of applying a constant voltage continuously to the enzyme-immobilized electrode, the response current was increased with time. GOD exhibits a high Km value and a slow reaction speed. Therefore, when a constant voltage is continuously applied, GOD and unreacted glucose are accumulated on the electrode surface with time. It is understood that, when a constant voltage is subsequently applied continuously in this state, glucose accumulated on the electrode surface reacts with GOD late, and as a result, the response current is increased with time. In contrast, when a pulse voltage is applied repeatedly to the enzyme-immobilized electrode using GOD, a response current obtained during application of the pulse voltage is measured, and hence, the response current comes to an almost constant value. In the pulse method, response voltages for individual pulses are each independently applied for only a predetermined application time. Accordingly, only a response current corresponding to a reaction of GOD and glucose within the predetermined application time is obtained. Because no response voltage is applied before the time at which glucose accumulated on the electrode surface reacts with GOD late, it is conceivable that the response current should come to an almost constant value. It should be noted that GDH exhibits a low Km value and a high reaction speed. Therefore, even if a constant voltage is applied continuously to the enzyme-immobilized electrode using GDH, the response current is not increased. The denaturation and deterioration of GDH through application of the voltage causes a decrease in response current depending on the continuous application time of the constant voltage.

Further, in Example 4, in the case of using GOD as a glucose oxidoreductase, an evaluation was made on the stability of the enzyme-immobilized electrode in the case of applying a pulse voltage repeatedly to the enzyme-immobilized electrode. The stability was evaluated as the sensitivity of the enzyme-immobilized electrode after continuous measurement of a response current by using a 600 mg/dL glucose solution. The setting of the voltage to be applied, and the applied voltage pattern are the same as those in the measurement of response characteristics of glucose.

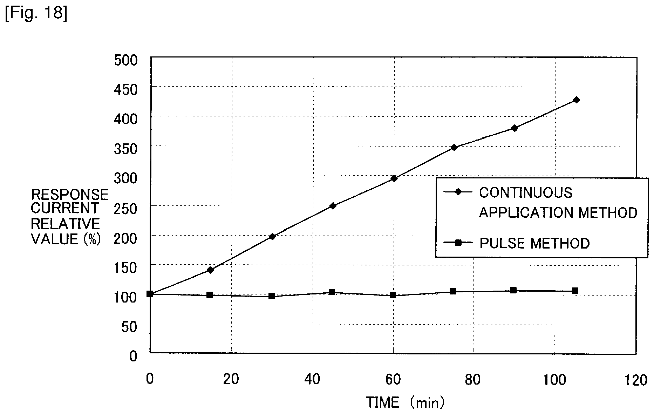

The voltage application time was set at 105 minutes, and the sensitivity of the enzyme-immobilized electrode was measured as a response current every 15 minutes. For comparison, an evaluation was made on the sensitivity in the case of applying a constant voltage of 600 mV continuously to the enzyme-immobilized electrode. FIG. 18 illustrates the sensitivity in those measurement methods expressed as a relative value (%) of the response current to the initial sensitivity (response current at 0 hour).

As illustrated in FIG. 18, in the method of applying a constant voltage continuously as a comparative example, the response current was increased with time. GOD exhibits a high Km value and a slow reaction speed. Therefore, when a constant voltage is continuously applied, GOD and unreacted glucose are accumulated on the electrode surface with time. It is understood that, when a constant voltage is subsequently applied continuously in this state, glucose accumulated on the electrode surface reacts with GOD late, and as a result, the response current is increased with time. In contrast, in the method of applying a pulse voltage repeatedly, an increase in response current was not observed even after a lapse of time, and it was revealed that the enzyme-immobilized electrode had satisfactory stability. In the pulse method, response voltages for individual pulses are each independently applied for only a predetermined application time. Accordingly, only a response current corresponding to a reaction of GOD and glucose within the predetermined application time is obtained. Because no response voltage is applied before the time at which glucose accumulated on the electrode surface reacts with GOD late, it is conceivable that the response current should come to an almost constant value.

### Example 5

In Example 5, studies were made on the dependence of the glucose concentration in the case of applying a constant voltage continuously to the enzyme-immobilized electrode, and the dependence of the glucose concentration in the case of applying a pulse voltage repeatedly to the enzyme-immobilized electrode. More specifically, in the case of applying a constant voltage continuously to the enzyme-immobilized electrode, a measurement was made on a response current in the case of changing the glucose concentration. Further, in the case of applying a pulse voltage repeatedly to the enzyme-immobilized electrode, a measurement was made on a response current in case of changing the glucose concentration. The response current was measured by adding glucose at a particular timing while allowing a stirring bar to rotate with a stirrer in a state where an enzyme-immobilized electrode (working electrode), a platinum electrode (counter electrode), and a silver/silver chloride electrode (reference electrode) were soaked in a container containing a phosphate buffer (100 mM, pH=7.0) and the stirring bar.

### (Production of enzyme-immobilized electrode)

Platinum was deposited on the surface of a polyimide base material by a sputtering method to a thickness of 30 nm, to thereby form a conductive layer on the surface of the polyimide base material. Then, the conductive layer was soaked in a GOD solution (1 mg/mL GOD aqueous solution) to form a GOD layer on the conductive layer. Thus, an enzyme-immobilized electrode was produced. The concentration of GOD in the GOD solution was 1,100 U/mL in terms of activity.

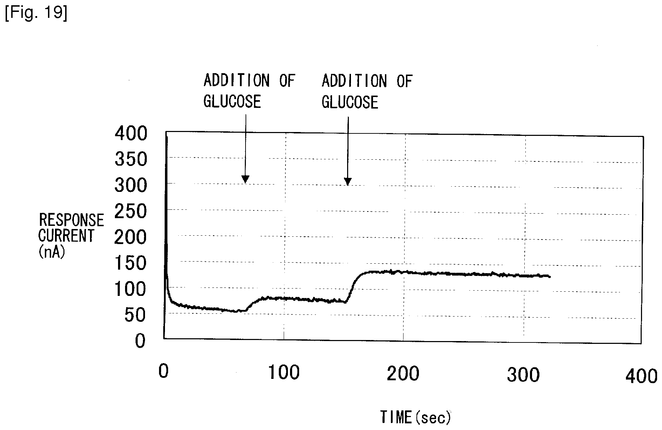

FIG. 19 is a graph illustrating a change in response current due to an increasing glucose concentration in the case of applying a constant voltage continuously to the enzyme-immobilized electrode. The addition of glucose was performed twice, and the glucose concentration was successively changed from 0 mg/dL to 100 mg/dL to 600 mg/dL. The response current was measured by applying a constant voltage of 600 mV continuously to the enzyme-immobilized electrode.

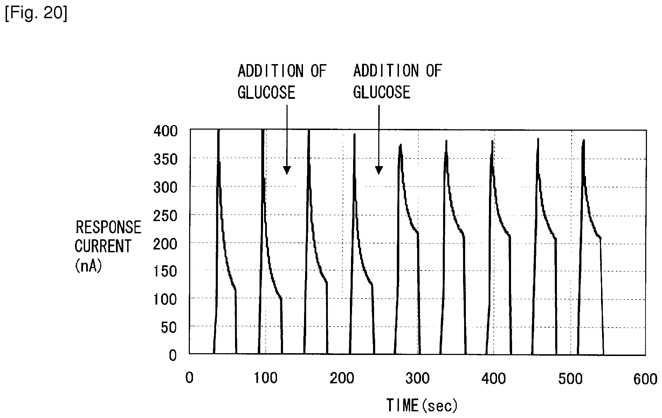

FIG. 20 is a graph illustrating a change in response current due to an increasing glucose concentration in the case of applying a pulse voltage repeatedly to the enzyme-immobilized electrode. A pulse voltage was applied twice and then glucose was added once, a pulse voltage was further applied twice and then glucose was added once, and a pulse voltage was still further applied five times. The glucose concentration was successively changed from 0 mg/dL to 100 mg/dL to 600 mg/dL by adding glucose twice. The response current was measured by setting the non-response voltage E1 to a constant voltage of 300 mV, and setting the response voltage E2 to a constant voltage of 600 mV. Further, the application time T1 of the non-response voltage E1 was set at 30 seconds, and the application time of the response voltage E2 was set at 30 seconds.

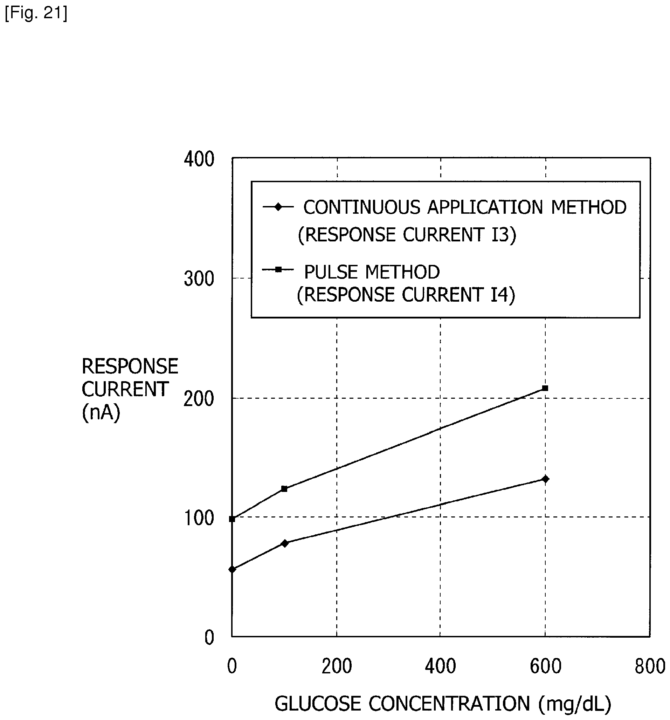

FIG. 21 is a graph illustrating the response current 13 in the case of applying a constant voltage continuously to the enzyme-immobilized electrode, and the response current I4 in the case of applying a pulse voltage repeatedly to the enzyme-immobilized electrode. As illustrated in FIG. 21, the response current 13 and the response current I4 were increased along with an increasing glucose concentration. Thus, it was confirmed that, even in the case of using GOD as a glucose oxidoreductase, the response current was increased through an increase in glucose concentration.

### Reference Signs List

1 glucose continuous measurement apparatus
4 glucose sensor
E1 non-reaction voltage (non-response voltage)
E2 reaction voltage (response voltage)
E3 standby voltage

## Claims

1. A method of continuously measuring a substance concentration based on a response when a voltage is applied to a sensor (4), the method comprising:
a response voltage application step of applying a response voltage at which a current attributed to a substance is obtained;
a non-response voltage application step of applying a non-response voltage at which a current attributed to a background component is obtained;
a comparing step of comparing the current obtained in the response voltage application step with a first threshold value, or comparing the current obtained in the non-response voltage application step with a second threshold value; and
a determining step of determining whether or not to calculate the substance concentration based on the result of the comparing step;
wherein the response voltage application step and the non-response voltage application step, and the respective obtaining of the current attributed to the substance and the current attributed to the background component, are performed alternately and repeatedly,
**characterised in that**,
the response voltage transitions to the non-response voltage without interruption, and the non-response voltage transitions to the response voltage without interruption.

2. The method of continuously measuring a substance concentration according to claim 1, wherein the substance concentration is calculated based on a difference between the current obtained in the response voltage application step and the current obtained in the non-response voltage application step.

3. The method of continuously measuring a substance concentration according to claim 2, wherein, when the voltage to be applied to the sensor (4) is increased, the non-response voltage is set in a range of -0.5 to +0.5 V with respect to an applied voltage at which the current attributed to the substance starts being obtained.

4. The method of continuously measuring a substance concentration according to claim 1, further comprising a standby step of applying a voltage smaller in an absolute value than the non-response voltage after performing the response voltage application step and the non-response voltage application step alternately a predetermined number of times.

5. The method of continuously measuring a substance concentration according to claim 1, further comprising a standby step of setting a circuit including the sensor (4) to become an open circuit after performing the response voltage application step and the non-response voltage application step alternately a predetermined number of times.

6. The method of continuously measuring a substance concentration according to any one of claims 1 to 5, wherein the sensor (4) is implanted in a body for use.

7. The method of continuously measuring a substance concentration according to any one of claims 1 to 6, wherein the sensor (4) includes an enzyme-immobilized electrode (42).

8. The method of continuously measuring a substance concentration according to any one of claims 1 to 7, wherein:
the substance comprises glucose; and
the sensor (4) comprises a glucose sensor.

9. The method of continuously measuring a substance concentration according to claim 8, wherein the glucose sensor (4) includes an electrode (42) with a glucose oxidoreductase immobilized thereon.

10. The method of continuously measuring a substance concentration according to claim 1, further comprising:
an intermediate voltage application step of applying an intermediate voltage,
wherein the intermediate voltage is a voltage smaller than the response voltage, but a voltage larger than the non-response voltage, and
a combination of the response voltage, the intermediate voltage and the non-response voltage are applied as a stepped pulse voltage.

## Patentansprüche

1. Verfahren zum kontinuierlichen Messen einer Substanzkonzentration auf der Basis einer Ansprechung, wenn eine Spannung an einen Sensor (4) angelegt wird, wobei das Verfahren Folgendes beinhaltet:
einen Ansprechspannungsanlegeschritt zum Anlegen einer Ansprechspannung, bei der ein einer Substanz zugeordneter Strom erhalten wird;
einen Nichtansprechspannungsanlegeschritt zum Anlegen einer Nichtansprechspannung, bei der ein einer Hintergrundkomponente zugeordneter Strom erhalten wird;
einen Vergleichsschritt zum Vergleichen des im Ansprechspannungsanlegeschritt erhaltenen Stroms mit einem ersten Schwellenwert oder Vergleichen des im Nichtansprechspannungsanlegeschritt erhaltenen Stroms mit einem zweiten Schwellenwert; und
einen Feststellungsschritt zum Feststellen auf der Basis des Ergebnisses des Vergleichsschritts, ob die Substanzkonzentration berechnet werden soll oder nicht;
wobei der Ansprechspannungsanlegeschritt und der Nichtansprechspannungsanlegeschritt und das jeweilige Erhalten des der Substanz zugeordneten Stroms und des der Hintergrundkomponente zugeordneten Stroms abwechselnd und wiederholt durchgeführt werden, **dadurch gekennzeichnet, dass**
die Ansprechspannung ohne Unterbrechung auf die Nichtansprechspannung übergeht und die Nichtansprechspannung ohne Unterbrechung auf die Ansprechspannung übergeht.

2. Verfahren zum kontinuierlichen Messen einer Substanzkonzentration nach Anspruch 1, wobei die Substanzkonzentration auf der Basis einer Differenz zwischen dem im Ansprechspannungsanlegeschritt erhaltenen Strom und dem im Nichtansprechspannungsanlegeschritt erhaltenen Strom berechnet wird.

3. Verfahren zum kontinuierlichen Messen einer Substanzkonzentration nach Anspruch 2, wobei, wenn die an den Sensor (4) anzulegende Spannung erhöht wird, die Nichtansprechspannung in einem Bereich von -0,5 bis +0,5 V mit Bezug auf eine angelegte Spannung eingestellt wird, bei der der der Substanz zugeordnete Strom beginnt erhalten zu werden.

4. Verfahren zum kontinuierlichen Messen einer Substanzkonzentration nach Anspruch 1, das ferner einen Standby-Schritt des Anlegens einer Spannung, deren Absolutwert kleiner als der der Nichtansprechspannung nach dem Durchführen des Ansprechspannungsanlegeschrittes und des Nichtansprechspannungsanlegeschrittes ist, abwechselnd eine vorbestimmte Anzahl von Malen beinhaltet.

5. Verfahren zum kontinuierlichen Messen einer Substanzkonzentration nach Anspruch 1, das ferner einen Standby-Schritt des Einstellens eines Schaltkreises inklusive des Sensors (4), so dass er zu einem offenen Schaltkreis wird, nach dem Durchführen des Ansprechspannungsanlegeschrittes und des Nichtansprechspannungsanlegeschrittes abwechselnd eine vorbestimmte Anzahl von Malen beinhaltet.

6. Verfahren zum kontinuierlichen Messen einer Substanzkonzentration nach einem der Ansprüche 1 bis 5, wobei der Sensor (4) in einem Körper zur Verwendung implantiert wird.

7. Verfahren zum kontinuierlichen Messen einer Substanzkonzentration nach einem der Ansprüche 1 bis 6, wobei der Sensor (4) eine enzymimmobilisierte Elektrode (42) beinhaltet.

8. Verfahren zum kontinuierlichen Messen einer Substanzkonzentration nach einem der Ansprüche 1 bis 7, wobei:
die Substanz Glucose umfasst; und
der Sensor (4) einen Glucosesensor umfasst.

9. Verfahren zum kontinuierlichen Messen einer Substanzkonzentration nach Anspruch 8, wobei der Glucosesensor (4) eine Elektrode (42) mit einer darauf immobilisierten Glucoseoxidoreduktase beinhaltet.

10. Verfahren zum kontinuierlichen Messen einer Substanzkonzentration nach Anspruch 1, das ferner Folgendes beinhaltet:
einen Zwischenspannungsanlegeschritt zum Anlegen einer Zwischenspannung,
wobei die Zwischenspannung eine Spannung ist, die niedriger als die Ansprechspannung, aber höher als die Nichtansprechspannung ist, und
eine Kombination aus Ansprechspannung, Zwischenspannung und Nichtansprechspannung als gestufte Pulsspannung angelegt wird.

## Revendications

1. Procédé de mesure continue d'une concentration de substance sur la base d'une réponse quand une tension est appliquée à un capteur (4), le procédé comprenant :
une étape d'application de tension de réponse, consistant à appliquer une tension de réponse à laquelle un courant attribué à une substance est obtenu ;
une étape d'application de tension de non-réponse, consistant à appliquer une tension de réponse à laquelle un courant attribué à une composante de fond est obtenu ;
une étape de comparaison, consistant à comparer à une première valeur-seuil le courant obtenu à l'étape d'application de tension de réponse, ou à comparer à une seconde valeur-seuil le courant obtenu à l'étape d'application de tension de non-réponse ; et
une étape de détermination, consistant à déterminer s'il faut calculer ou non la concentration de substance sur la base du résultat de l'étape de comparaison ;
l'étape d'application de tension de réponse, l'étape d'application de tension de non-réponse et l'obtention respective du courant attribué à la substance et du courant attribué à la composante de fond étant réalisées en alternance et de façon répétée,
le procédé étant **caractérisé en ce que** :
la tension de réponse change pour la tension de non-réponse sans interruption et la tension de non-réponse change pour la tension de réponse sans interruption.

2. Procédé de mesure continue d'une concentration de substance selon la revendication 1, dans lequel la concentration de substance est calculée sur la base d'une différence entre le courant obtenu à l'étape d'application de tension de réponse et le courant obtenu à l'étape d'application de tension de non-réponse.

3. Procédé de mesure continue d'une concentration de substance selon la revendication 2, dans lequel, quand la tension à appliquer au capteur (4) est augmentée, la tension de non-réponse est fixée de l'ordre de -0,5 à +0,5 V par rapport à une tension appliquée à laquelle le courant attribué à la substance commence à être obtenu.

4. Procédé de mesure continue d'une concentration de substance selon la revendication 1, comprenant en outre une étape d'attente consistant à appliquer une tension en valeur absolue inférieure à la tension de non-réponse après la réalisation de l'étape d'application de tension de réponse et l'étape d'application de tension de non-réponse en alternance et un nombre prédéterminé de fois.

5. Procédé de mesure continue d'une concentration de substance selon la revendication 1, comprenant en outre une étape d'attente consistant à régler un circuit comprenant le capteur (4) de sorte que ledit circuit devienne un circuit ouvert après la réalisation de l'étape d'application de tension de réponse et l'étape d'application de tension de non-réponse en alternance et un nombre prédéterminé de fois.

6. Procédé de mesure continue d'une concentration de substance selon l'une quelconque des revendications 1 à 5, dans lequel le capteur (4) est mis en œuvre dans un corps en vue de son utilisation.

7. Procédé de mesure continue d'une concentration de substance selon l'une quelconque des revendications 1 à 6, dans lequel le capteur (4) comprend une électrode à immobilisation enzymatique (42).

8. Procédé de mesure continue d'une concentration de substance selon l'une quelconque des revendications 1 à 7, dans lequel :
la substance comprend du glucose ; et
le capteur (4) comprend un capteur de glucose.

9. Procédé de mesure continue d'une concentration de substance selon la revendication 8, dans lequel le capteur de glucose (4) comprend une électrode (42) avec une glucose oxydoréductase immobilisée dessus.

10. Procédé de mesure continue d'une concentration de substance selon la revendication 1, comprenant en outre :
une étape d'application de tension intermédiaire, consistant à appliquer une tension intermédiaire,
la tension intermédiaire étant une tension inférieure à la tension de réponse, mais une tension supérieure à la tension de non-réponse, et
une combinaison de la tension de réponse, de la tension intermédiaire et de la tension de non-réponse étant appliquée en guise de tension puisée échelonnée.
